# EUROPÄISCHE PATENTSCHRIFT

(11) **EP 0 533 210 B1**
(45) Veröffentlichungstag und Bekanntmachung des Hinweises auf die Patenterteilung: **02.05.1997**
(21) Anmeldenummer: 92117249.0
(22) Anmeldetag: 28.01.1991
(51) Int. Cl.: A61K 38/48

(54) **Protein C-haltige pharmazeutische Präparation als Mittel gegen Entzündungen**
Protein C containing pharmaceutical preparations as anti-inflammatory agent
Préparations pharmaceutiques contenant la protéine C comme agent anti-inflammatoire

(30) Priorität: 16.08.1990 AT 1697/90
(43) Veröffentlichungstag der Anmeldung: 24.03.1993
(62) Teilanmeldung aus: 91890012.7
(73) Patentinhaber: IMMUNO Aktiengesellschaft, A-1221 Wien (AT)
(72) Erfinder: Eibl, Johann, Dr., A-1180 Wien (AT); Pichler, Ludwig, Prof. Dr., A-1040 Wien (AT); Schwarz, Hans-Peter, Doz. Dr., A-1180 Wien (AT)
(74) Vertreter: Wolfram, Gustav, Dipl.-Ing.

(56) Entgegenhaltungen:
- EP-A- 0 326 014
- BIOLOGICAL ABSTRACTS, vol. 85, no. 4, 1985, Philadelphia, PA, US; abstract no. 37736; K. ZURBORN ET AL: 'Immunological and functional evaluation of Protein C in several internal diseases'
- CHEMICAL ABSTRACTS, vol. 95, no. 25, 21. Dezember 1981, Columbus, Ohio, US; abstract no. 218287; P. COMP AND C. ESMON: 'Generation of fibrinolytic activity by infusion of activated Protein C into dogs', Seite 347
- SEMINARS IN THROMBOSIS AND HEMOSTASIS, Bd. 14, Nr. 2, April 1988, NEW YORK, Seiten 210-215; N. ESMON AND C. ESMON: 'Protein C and the endothelium'
- JOURNAL OF CLINICAL INVESTIGATION, Bd. 79, März 1987, Seiten 918-925; F. TAYLOR ET AL: 'Protein C prevents the coagulopathic and lethal effects of Escherichia coli infusion in the baboon'

## Beschreibung

Die Erfindung betrifft eine neue Verwendung von Protein C zur Herstellung eines Arzneimittels zur Behandlung von Entzündungen.

Protein C ist ein Vitamin-K-abhängiges Glykoprotein, das in der Leber synthetisiert wird und im Plasma in einer Konzentration von 4 µg/ml als inaktives Zymogen zirkuliert. Es wird an der Gefäßwandoberfläche (Endothel) durch den Thrombin-Thrombomodulinkomplex in die aktive Serinprotease (aktiviertes Protein C) übergeführt. Es ist bekannt, daß aktiviertes Protein C profibrinolytische Eigenschaften hat. Es wirkt auch antikoagulatorisch, weil es den Faktor Va, den Kofaktor für die Faktor Xa-induzierte Prothrombinaktivierung (Thrombinbildung) und den Faktor VIIIa, den Kofaktor für die Faktor IXa-induzierte Faktor X-Aktivierung, durch Proteolyse abbaut.

Die Aktivierung von Protein C in vivo stellt eine negative Feedback-Reaktion der Thrombingenerierung dar. Um optimale biologische Aktivität zu entfalten, ist ein Kofaktor (Protein S) notwendig.

In der österreichischen Patentanmeldung AT 1551/89 (EP-A-406216) ist eine pharmazeutische Präparation beschrieben, die Protein C enthält und zur Behandlung bzw. Verhinderung von Thrombosen und thromboembolischen Komplikationen eingesetzt werden kann.

Da sowohl das Protein C als auch das aktivierte Protein C als körpereigene Proteine anzusehen sind, besitzen pharmazeutische Präparationen auf Basis dieser Proteine den großen Vorteil, daß sie praktisch ohne Nebenwirkungen verabreicht werden können.

In Biol. Abstracts 85(4) (1985); Nr.37736 wird eine neuartige Protein C-Bestimmungsmethode beschrieben, wobei sowohl die Aktivität von Protein C (nach Aktivierung mit Schlangengift) als auch die Gesamtkonzentration von PC und aPC bestimmt wird.

In Seminars in Thrombosis and Hemostatis 14 (2) (1988), Seiten 210-215, wird darauf hingewiesen, daß der Protein C-Antikoagulationsweg in der Wechselwirkung zwischen Blutgerinnung und Entzündungssystemen eine wichtige Rolle spielt.

In Journal of Clinical Investigation 79 (1987), Seiten 918-925 wird die Applikation von aktiviertem Protein C in Pavianen, die einen E. Coli-Schock erhalten haben, beschrieben.

Die Erfindung stellt sich die Aufgabe, das therapeutische Anwendungsgebiet von Protein C bzw. von aktiviertem Protein C zu vergrößern und sie besteht darin, daß Protein C oder aktiviertes Protein C zur Herstellung einer pharmazeutischen Präparation zur Verhinderung bzw. Behandlung akuter oder chronischer entzündlicher Erkrankungen verwendet wird.

Die parallele Anmeldung EP 91890912 (EP-A-471660) beruht auf der Erkenntnis, daß die durch entzündliche Prozesse induzierte, erhöhte Schmerzempfindung mittels Protein C herabgesetzt werden kann.

Die parallele Anmeldung EP92117248 (EP-A-533209) betrifft die Verwendung von Protein C als beta-Sympathomimetikum.

Es hat sich weiters gezeigt, daß Protein C oder aktiviertes Protein C imstande ist, die Adhäsion von Leukozyten an den Gefäßwänden zu verhindern und das Wandern der Leukozyten durch die Gefäßwände zu hemmen. Beide Proteine unterbinden die Aktivierung der Leukozyten und verhindern die Komplementaktivierung. Diese entzündlichen Erkrankungen, für welche Protein C erfindungsgemäß verwendet wird, können post-traumatisch, post-operativ oder im Rahmen primärer und sekundärer maligner Erkrankungen auftreten.

Die pharmazeutische Präparation eignet sich insbesondere zur Behandlung rheumatoider Arthritis, Myositis, Gastritis oder Kolitis, zur Behandlung von Entzündungen im Urogenitaltrakt, sowie zur Behandlung entzündlicher Prozesse, die im Rahmen akuter oder chronischer Transplantatabstoßung auftreten.

Die Erfindung betrifft auch die Verwendung von Protein C zur Herstellung eines pharmazeutischen Kombinationspräparates mit mindestens einem Antiphlogistikum. Es hat sich gezeigt, daß ein derartiges Kombinationspräparat synergistische Effekte aufweist.

Weiters betrifft die Erfindung die Verwendung von Protein C oder aktiviertem Protein C zur Herstellung von Präparationen zur Normalisierung einer gesteigerten Gefäßpermeabilität und zur Verhinderung von Permeabilitätsschäden der Gefäßwand.

Die Gewinnung von Protein C und von aktiviertem Protein C, sowie die Prüfung auf ihre erfindungsgemäße Wirkung werden nachfolgend beschrieben.

### Gewinnung von Protein C

Hochreines Protein C wurde aus einer rohen Protein C-Fraktion gewonnen, die aus kommerziell erhältlichem Prothrombinkomplex-Konzentrat hergestellt wurde. Die Reinigung erfolgte affinitätschromatographisch mittels monoklonaler Antikörper. Monoklonale Anti-Protein C-Antikörper wurden wie folgt hergestellt:

BALB/C-Mäuse wurden mit 100 µg menschlichem Protein C in zweiwöchigen Abständen durch intraperitoneale Injektion immunisiert. Nach sechs Wochen wurden noch einmal 50 µg des menschlichen Protein C injiziert und drei Tage später die Fusion vorgenommen. Die Myelomzellinie (P3-X-63-AG8-653, 1,5 x 10⁷ Zellen) wurde vermischt mit 1,7 x 10⁸ Milzzellen von einer Maus, und die Fusion nach modifizierter Köhler & Milstein-Methode unter Verwendung von PEG 1500 durchgeführt (Köhler G., Milstein C., Nature 256(1975)495-497).

Positive Klone, getestet mittels eines ELISA, wurden zweimal subgeklont. Aszitesproduktion erfolgte durch Injektion von 5 x 106 Hybridomzellen je BALB/C-Maus zwei Wochen nach Pristan-Behandlung.

Das Immunglobulin wurde aus Aszites durch Ammoniumsulfatpräzipitation und anschließende Chromatographie mittels QAE-Sephadex und darauffolgend chromatographie an Sephadex G200 gereinigt. Um das Risiko der Übertragung muriner Viren zu reduzieren, wurde der Antikörper vor der immobilisierung noch einem Virusinaktivierungsschritt unterworfen. Die so erhaltenen monoklonalen Antikörper gegen Protein C wurden an CNBR-Sepharose 4B (Pharmacia) gekoppelt. Für die Reinigung des Protein C mittels Affinitätschromatographie wurden folgende Puffer verwendet:

Als Adsorptionspuffer: 20 mM Tris, 2 mM EDTA, 0,25 M NaCl und 5 mM Benzamidin;
als Waschpuffer wurde verwendet: 20 mM Tris, 1 M NaCl, 2 mM Benzamidin, 2 mM EDTA; der pH-Wert betrug 7,4;
als Elutionspuffer wurde verwendet: 3 M NaSCN, 20 mM Tris, 1 M NaCl, 0,5 mM Benzamidin, 2 mM EDTA.

Im einzelnen: Das Prothrombinkomplex-Konzentrat wurde im Adsorptionspuffer aufgelöst, wobei etwa 10 g des Prothrombinkomplex-Konzentrates für eine 20 ml monoklonale Antikörpersäule zur Verwendung kamen. Anschließend wurde das aufgelöste Prothrombinkomplex-Konzentrat filtriert, bei 20.000 rpm 15 min lang zentrifugiert und durch ein 0,8 µm Filter sterilfiltriert. Das sterilfiltrierte und gelöste Prothrombinkomplex-Konzentrat wurde auf die Säule aufgetragen mit einer Flußrate von 10 ml/h. Anschließend wurde die Säule mit dem Waschpuffer proteinfrei gewaschen und schließlich das gebundene Protein C mit dem Elutionspuffer bei einer Flußrate von 5 ml/h eluiert und die Fraktionen gesammelt. Das eluierte Protein C wurde gegen einen Puffer (0,2 M Tris, 0,15 M Glycin und 1 mM EDTA, pH 8,3) dialysiert. Der Protein C-Gehalt wurde antigenmäßig mittels der Methode nach Laurell und aktivitätsmäßig nach Protac-Aktivierung bestimmt.

Das erhaltene Protein C-Eluat wurde in folgender Weise zu einer pharmazeutisch applizierbaren Präparation fertiggestellt:

Das Eluat wurde zuerst einem Ultrafiltrations- und einem Diafiltrationsschritt unterworfen. Für die Diafiltration wurde ein Puffer mit einem pH-Wert von 7,4 verwendet, der pro Liter 150 mmol NaCl und 15 mmol Trinatriumcitrat.2H₂O enthielt. Das erhaltene Filtrat wurde gefriergetrocknet und durch eine einstündige Dampfbehandlung bei 800°C ± 5°C und 1375 ± 35 mbar virusinaktiviert.

Das lyophilisierte virusinaktivierte Material wurde sodann in einer sterilen isotonen NaCl-Lösung gelöst und mittels Ionenaustauschchromatographie an Q-Sepharose wurden etwaig vorhandene Antikörper bzw. Serumamyloid P entfernt. Die gereinigte Lösung wurde durch einen weiteren Ultrafiltrations- und Diafiltrationsschritt konzentriert. Danach wurden zur erhaltenen Lösung pro Liter 10 g Albumin, 150 mmol NaCl und 15 mmol Trinatriumcitrat zugegeben. Der pH-Wert der Lösung betrug 7,5. Maus-Immunglobulin sowie die Faktoren II, VII, IX und X konnten nicht nachgewiesen werden. Anschließend wurde die Lösung sterilfiltriert, abgefüllt und lyophilisiert. Die spezifische Aktivität betrug 14 Einheiten Protein C/mg. Eine Einheit Protein C Aktivität ist definiert als die Protein-C-Aktivität in 1 ml Normalplasma und wird gegen den ersten internationalen Standard von Protein C kalibriert. Als Aktivitätstest wurde ein amidolytischer Test verwendet, wobei das Protein C mittels Protac (Fa. Pentapharm) aktiviert wurde.

### Gewinnung von aktiviertem Protein C

Die Aktivierung des gereinigten Protein C erfolgte dadurch, daß 70 ml Thrombin (500 NIH Einheiten/ml, etwa 2000 NIH Einheiten/mg Protein entsprechend) an CNBR Sepharose 4B (Pharmacia) gekoppelt wurden, worauf Protein C mit dem Thrombingel in einem Verhältnis von etwa 6 Einheiten Protein C zu 1 Einheit Thrombin bei 370 c gemischt und unter kontinuierlichem Schütteln 3 h in Reaktion belassen wurden. Die Protein C-Aktivität wurde anschließend mit chromogenem Substrat (5 2366) bestimmt. Das aktivierte Protein C wurde anschließend sterilfiltriert und durch Abfüllung und Lyophilisation zu einer pharmazeutischen Präparation fertiggestellt.

### Antinociceptive Wirkung

Die antinociceptive Wirkung wurde an der entzündeten Rattenpfote durch Bestimmung der Schmerzschwelle, mit und ohne Gabe von Protein C/aktiviertem Protein C, nachgewiesen.

Die Schmerzschwelle wurde analog der von Randall und Selitto beschriebenen Methode gemessen (Randall LO and Selitto JJ: A method for measurement of analgesic activity on inflamed tissue, Arch. Int. Pharmacodyn. 111, 409-419, 1957). Dazu wurde ein Quecksilbermanometer mit einer 10 ml Spritze verbunden, deren Kolben mit einem kurzen, geschoßförmigen Zapfen ausgerüstet war. Durch diese Spitze wurde steigender Druck auf die Rattenpfote ausgeübt (20 mmHg/Sekunde). Die Schmerzschwelle wurde angegeben als jener Druck in mmHg, der notwendig ist, um die Fluchtreaktion der Pfote auszulösen.

Zur Untersuchung wurden weibliche Sprague-Dawley Ratten mit einem Gewicht zwischen 250 und 350 g verwendet. Die Entzündung wurde mittels 3 mg Carrageenan (Sigma, C-1013), aufgelöst in 100 µl isotoner Kochsalzlösung, durch intraplantare Injektion in die Rattenpfote ausgelöst.

Die Schmerzschwelle wurde vor der Injektion (Kontrollwert) und stündlich bis zu 6 Stunden nach der Injektion von Carrageenan gemessen. Die Schmerzschwelle, die nach der Carrageenaninjektion gemessen wurde, wurde ausgdrückt in Prozent des Kontrollwertes.

Protein C oder aktiviertes Protein C wurden intravenös in eine Schwanzvene unmittelbar nach der Carrageenaninjektion injiziert. Das Injektionsvolumen betrug 2 ml/kg.

Es wurde gezeigt, daß die unter Carrageenan-Einfluß reduzierte Schmerzschwelle durch Gabe von Protein C angehoben wird (antinociceptiver Effekt). Dazu wurde die antinociceptive Wirkung bie Protein C-Dosen zu 8 E/kg (10 Tiere), zu 25 E/kg (15 Tiere), zu 80 E/kg (20 Tiere), zu 250 E/kg (20 Tiere) und zu 800 E/kg (15 Tiere) bestimmt. Der antinociceptive Effekt von Protein C war dosisabhängig und signifikant (p < 0,01 für 80 Einheiten/kg; p < 0,001 für 250 und 800 Einheiten/kg).

Auch subkutan gegeben läßt sich dieser Effekt für Protein C nachweisen.

Es konnte weiters nachgewiesen werden, daß auch aktiviertes Protein C in einer Dosis von 250 E/kg 2 Stunden nach der intraplantaren Injektion von Carrageenan eine antinociceptive Wirkung besitzt. Aktiviertes Protein C in einer Dosis von 250 E/kg intravenös 10 Minuten vor der Carrageenan-ausgelösten Entzündung hat ebenfalls einen antinociceptiven Effekt, der 2 Stunden anhält. Der antinociceptive Effekt von aktiviertem Protein C ist ebenfalls dosisabhängig.

Histologische Untersuchungen der Rattenpfoten, die mit Protein C oder aktiviertem Protein C behandelt wurden, zeigten eine verringerte perivaskuläre Leukozytenzahl im Vergleich zu unbehandelten Tieren.

Es hat sich weiters gezeigt, daß der nicht selektive β-Adrenozeptorenblocker Propranolol die antinozizeptive Wirkung von Protein C (800 E/kg) aufhebt. Die Protein C-antagonisierende Wirkung von Propranolol konnte im Dosisbereich von 0,03 bis 1 mg/kg i.v. nachgewiesen werden (siehe Tabelle 1).

**Tabelle 1**

| Propranolol (Ergebnisse in % der Schmerzschwelle, gemessen 3 h nach Carrageenin im Rattenpfotenmodell wie oben beschrieben) | | |
|---|---|---|
| | | Anzahl |
| Carrageenin allein | 21 ± 3 % | 15 |
| Carrageenin + Protein C (800 E/kg) | 60 ± 6 % | 15 |
| Carrageenin + Protein C + Propranolol (1 mg/kg) | 28 ± 5 % | 10 |

Dieser Antagonismus ist aber nicht auf Propranolol beschränkt, sondern auch andere β-blockierende Substanzen antagonisieren dosisabhängig die Protein C-Wirkung, wie in Tabelle 2 für Pindolol gezeigt.

**Tabelle 2**

| Pindolol (Ergebnisse in % der Schmerzschwelle, gemessen 3 h nach Carrageenin im Rattenpfotenmodell wie oben beschrieben) | | |
|---|---|---|
| | | Anzahl |
| Carrageenin allein | 22 ± 1,3 % | 10 |
| Carrageenin + Protein C (800 E/kg) | 65 ± 2,0 % | 10 |
| Carrageenin + Protein C + Pindolol (0,3 mg/kg) | 24 ± 2,0 % | 5 |

Nach Ausschaltung des sympathischen Nervensystems durch chemische Sympathektomie mit Reserpin (7,5 mg/kg i.p., 18 bis 24 h) und alpha-Methyl-p-tyrosin (250 mg/kg i.p., 5 h) ist Protein C stark antinozizeptiv wirksam. Aber auch an diesen sympathektomierten Tieren antagonisiert Pindolol den Effekt von Protein C völlig (Tabelle 3). Somit kann eine Beteiligung des präsynaptischen Sympathikus an der Wirkung von Protein C ausgeschlossen werden. Der Effekt von Protein C ist daher durch direkte Wirkung an postsynaptischen β-Adrenorezeptoren bedingt.

**Tabelle 3**

| Reserpin und alpha-Methyl-p-tyrosin (Ergebnisse in % der Schmerzschwelle, gemessen 3 h nach Carrageenin im Rattenpfotenmodell wie oben beschrieben) Ausgangswerte (20) bei: 39 ± 1,2 mmHg | | |
|---|---|---|
| | | Anzahl |
| NaCl Kontr. | 61,0 ± 6,8 % | 5 |
| Carrageenin allein | 34,0 ± 10,3 % | 5 |
| Carrageenin + Protein C (800 E/kg) | 106,7 ± 18,1 % | 5 |
| Carrageenin + Protein C + Pindolol (1 mg/kg i.v.) | 33,3 ± 4,6 % | 5 |

Der Effekt von Protein C ist durch β-Sympathomimetika nachzuahmen. Fenoterol, entweder mit 5 mg/kg zu Versuchsbeginn oder mit 3 mg/kg zum Zeiptunkt "3 h" i.v. injiziert, führt zu deutlicher Antinonzizeption. Somit führt die Stimulierung postsynaptischer β-Adrenozeptoren zu antinozizeptiver Wirkung.

Die biochemische Wirkung des β-sympathomimetischen Effektes von Protein C wird durch Bindung an adrenergen β-Rezeptoren und Stimulierung des membrangebundenen Enzyms Adenylcyclase, welches die intrazelluläre Bildung von cAMP aus ATP katalysiert, hervorgerufen. cAMP aktiviert Protein-phosphokinasen, die ihrerseits durch Phosphorylierung inaktive in aktive Enzyme überführen. Auf diese Weise werden z.B. Lipolyse und Glukogenolyse β-sympathomimetisch gesteigert.

Die physiologischen Auswirkungen von Protein C aufgrund seiner β-sympathomimetischen Wirkung sind vielfältig. Die Erhöhung von cAMP in den Thrombozyten führt zu einer Hemmung der Thrombozytenfunktion und zu einer Hemmung von Thrombinbindung an die Thrombozytenoberfläche. Somit kann Protein C zum Einsatz kommen, um thrombozytenabhängige Blutgerinnselbildungen, besonders im arteriellen Gefäßsystem durch Bindung an die thrombozytären β-Adrenozeptoren zu verhindern. Die β-adrenozeptor-positive Wirkung von Protein C hat am Herzen einen positiv inotropen Effekt und wirkt auf die glatte Muskulatur (z.B. an den Bronchiolen) erschlaffend. Aufgrund der β-sympathomimetischen Wirkung am Gefäßsystem kann Protein C therapeutisch bei peripheren Durchblutungsstörungen eingesetzt werden. Es hat sich auch gezeigt, daß die intrakoronare Gabe von aktiviertem Protein C bei Hunden zu einer Steigerung des koronaren Blutflusses aufgrund der vasodilatatorischen Wirkung führt. Die vasodilatatorische Wirkung von Protein C kann zur Behandlung der Hypertonie herangezogen werden. Der β-sympathomimetischen Wirkung von Protein C kann auch eine entzündungshemmende Eigenschaft zugeschrieben werden. Die β-sympathomimetische Wirkung von Protein C führt zu einer Hemmung der Histaminfreisetzung in der Lunge. Durch die β-sympathomimetische Wirkung von Protein C kann Protein C als Tokolytikum Anwendung finden.

## Patentansprüche

1. Verwendung von Protein C zur Herstellung einer pharmazeutischen Präparation zur Verhinderung bzw. Behandlung akuter oder chronischer entzündlicher Erkrankungen.

2. Verwendung von Protein C nach Anspruch 1 zur Herstellung einer Präparation zur Verhinderung bzw. Behandlung entzündlicher Prozesse, die post-traumatisch, post-operativ oder im Rahmen primärer und sekundärer maligner Erkrankungen auftreten.

3. Verwendung von Protein C nach Anspruch 1 zur Herstellung einer Präparation zur Verhinderung bzw. Behandlung rheumatoider Arthritis, Myositis, Gastritis oder Kolitis oder zur Behandlung von Entzündungen im Urogenitaltrakt.

4. Verwendung von Protein C nach Anspruch 1 zur Herstellung einer Präparation zur Verhinderung bzw. Behandlung entzündlicher Prozesse, die im Rahmen akuter oder chronischer Transplantatabstoßung auftreten.

5. Verwendung von Protein C zur Herstellung eines pharmazeutischen Kombinationspräparates mit mindestens einem Antiphlogistikum.

6. Verwendung von Protein C oder aktiviertem Protein C zur Herstellung einer pharmazeutischen Präparation zur Normalisierung einer gesteigerten Gefäßpermeabilität bzw. zur Verhinderung von Permeabilitätsschäden der Gefäßwand.

## Claims

1. The use of protein C for preparing a pharmaceutical preparation for the prevention and treatment of acute or chronic inflammatory diseases.

2. The use of protein C according to claim 1 for preparing a preparation for the prevention and treatment of inflammatory processes occurring posttraumatically, postoperatively or within the scope of primary and secondary malignant diseases.

3. The use of protein C according to claim 1 for preparing a preparation for the prevention and treatment of rheumatoid arthritis, myositis, gastritis or colitis or for the treatment of inflammations in the urogenital tract.

4. The use of protein C according to claim 1 for preparing a preparation for the prevention and treatment of inflammatory processes associated with acute or chronic graft rejection.

5. The use of protein C for preparing a pharmaceutical combination preparation including at least one antiphlogistic agent.

6. The use of protein C or activated protein C for preparing a pharmaceutical preparation for normalizing an elevated vascular permeability and for preventing permeability defects at a vessel wall.

## Revendications

1. Utilisation de protéine C pour la production d'une préparation pharmaceutique destinée à prévenir ou traiter des pathologies inflammatoires aiguës ou chroniques.

2. Utilisation de protéine C selon la revendication 1 pour la production d'une préparation destinée à prévenir ou traiter des processus inflammatoires post-traumatiques, post-opératoires ou survenant dans le cadre de pathologies malignes primaires et secondaires.

3. Utilisation de protéine C selon la revendication 1 pour la production d'une préparation destinée à prévenir ou traiter la polyarthrite rhumatoïde, la myosite, la gastrite ou la colite ou à traiter des inflammations de l'appareil urogénital.

4. Utilisation de protéine C selon la revendication 1 pour la production d'une préparation destinée à prévenir ou traiter des processus inflammatoires survenant dans le cadre du rejet aigu ou chronique de greffes.

5. Utilisation de protéine C pour la production d'une association pharmaceutique comportant au moins un anti-inflammatoire.

6. Utilisation de protéine C ou de protéine C activée pour la production d'une préparation pharmaceutique destinée à normaliser l'hyperperméabilité vasculaire ou à prévenir les lésions de perméabilité de la paroi vasculaire.
